# EUROPÄISCHE PATENTSCHRIFT

(11) **EP 1 200 423 B1**
(45) Veröffentlichungstag und Bekanntmachung des Hinweises auf die Patenterteilung: **01.10.2003**
(21) Anmeldenummer: 00949242.2
(22) Anmeldetag: 03.07.2000
(51) Int. Cl.: C07D 405/10, C07D 409/10, C07D 411/10, A01N 43/54

(54) **SUBSTITUIERTE PHENYLURACILE**
SUBSTITUTED PHENYL URACILS
PHENYLURACILES SUBSTITUES

(30) Priorität: 14.07.1999 DE 19932813
(43) Veröffentlichungstag der Anmeldung: 02.05.2002
(73) Patentinhaber: Bayer CropScience AG, 40789 Monheim (DE)
(72) Erfinder: ANDREE, Roland, 40764 Langenfeld (DE); DREWES, Mark-Wilhelm, 40764 Langenfeld (DE); DAHMEN, Peter, 41470 Neuss (DE); FEUCHT, Dieter, 40789 Monheim (DE); PONTZEN, Rolf, 42799 Leichlingen (DE)
(86) Internationale Anmeldenummer: EP0006179
(87) Internationale Veröffentlichungsnummer: WO01005785

(56) Entgegenhaltungen:
- EP-A- 0 542 685
- WO-A-95/06641

## Beschreibung

Die Erfindung betrifft neue substituierte Phenyluracile, Verfahren zu ihrer Herstellung und ihre Verwendung als Herbizide.

Bestimmte substituierte Aryluracile sind bereits aus der (Patent-)Literatur bekannt (vgl. EP-A-255 047, EP-A-260 621, EP-A-408 382, EP-A-438 209, EP-A-473 551, EP-A-517 181, EP-A-542 685, EP-A-563 384, WO-A-91/00278, WO-A-91/07393, WO-A-93/06090, WO-A-93/14073, WO-A-95/06641, WO-A-97/45418, US-A-49 79 982, US-A-50 84 084, US-A-51 27 935, US-A-51 54 755, US-A-51 69 430, US-A-54 86 610, US-A-53 56 863). Diese Verbindungen haben jedoch bisher keine besondere Bedeutung erlangt.

Es wurden nun neue substituierte Phenyluracile der allgemeinen Formel (I) 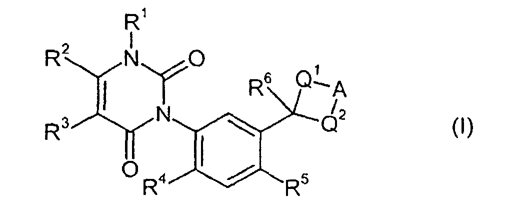 in welcher
- A: für jeweils gegebenenfalls durch Cyano, Halogen oder jeweils gegebenenfalls durch Halogen oder C₁-C₄-Alkoxy substituiertes C₁-C₄-Alkyl, C₁-C₄-Alkoxycarbonyl oder C₂-C₄-Alkenyl substituiertes oder durch O (Sauerstoff) unterbrochenes Alkandiyl oder Alkendiyl mit jeweils 2 bis 5 Kohlenstoffatomen, oder für Cyclohexan -1,2-diyl oder 1 2-Phenylen steht,
- Q¹: für O (Sauerstoff) oder S (Schwefel) steht,
- Q²: für O (Sauerstoff) oder S (Schwefel) steht,
- R¹: für Wasserstoff, Amino oder gegebenenfalls durch Cyano, Halogen oder C₁-C₄-Alkoxy substituiertes Alkyl mit 1 bis 4 Kohlenstoffatomen steht,
- R²: für gegebenenfalls durch Halogen substituiertes Alkyl mit 1 bis 4 Kohlenstoffatomen steht,
- R³: für Wasserstoff, Halogen oder gegebenenfalls durch Halogen substituiertes Alkyl mit 1 bis 4 Kohlenstoffatomen steht,
- R⁴: für Wasserstoff, Cyano, Carbamoyl, Thiocarbamoyl oder Halogen steht,
- R⁵: für Cyano, Carbamoyl, Thiocarbamoyl oder jeweils gegebenenfalls durch Halogen substituiertes Alkyl oder Alkoxy mit jeweils 1 bis 4 Kohlenstoffatomen steht, und
- R⁶: für Wasserstoff oder Alkyl mit 1 bis 4 Kohlenstoffatomen steht,
gefunden.

Bevorzugte Substituenten bzw. Bereiche der in den oben und nachstehend aufgeführten Formeln vorhandenen Reste werden im folgenden definiert.
- A: steht bevorzugt für jeweils gegebenenfalls durch Cyano, Fluor, Chlor, Brom, oder jeweils gegebenenfalls durch Fluor und/oder Chlor, Methoxy oder Ethoxy substituiertes Methyl, Ethyl, n- oder i-Propyl, Methoxycarbonyl, Ethoxycarbonyl, n- oder i-Propoxycarbonyl, Ethenyl oder Propenyl substituiertes oder durch O (Sauerstoff) unterbrochenes Ethan-1,2-diyl (Dimethylen), Propan-1,3-diyl (Trimethylen), Etlien- 1 ,2-diyl oder Proper-1.3-diyl, oder für Cyclohexan-1,2-(diyl oder 1,2-Phenylen.
- Q¹: steht bevorzugt für O (Sauerstoff).
- Q²: steht bevorzugt für O (Sauerstoff).
- R¹: steht bevorzugt für Wasserstoff, Amino oder jeweils gegebenenfalls durch Cyano, Fluor, Chlor, Methoxy oder Ethoxy substituiertes Methyl, Ethyl, n-oder i-Propyl.
- R²: stcht bevorzugt für jeweils gegebenenfalls durch Fluor und/oder Chlor substituiertes Methyl, Ethyl, n- oder i-Propyl.
- R³: steht bevorzugt für Wasserstoff, Fluor, Chlor oder jeweils gegebenenfalls durch Fluor und/oder Chlor substituiertes Methyl oder Ethyl.
- R⁴: steht bevorzugt für Wasserstoff, Cyano, Carbamoyl, Thiocarbamoyl, Fluor, Chlor oder Brom.
- R⁵: steht bevorzugt für Cyano, Carbamoyl, Thiocarbamoyl oder jeweils gegebenenfalls durch Fluor und/oder Chlor substituiertes Methyl, Ethyl, Methoxy oder Ethoxy.
- R⁶: steht bevorzugt für Wasserstoff, Methyl oder Ethyl.
- A: steht besonders bevorzugt für jeweils gegebenenfalls durch Fluor, Chlor, Methyl, Ethyl, Methoxycarbonyl oder Ethoxycarbonyl substituiertes Ethan-1,2-diyl (Dimethylen), Propan-1,3-diyl (Trimethylen) oder Ethen- 1 ,2-diyl, oder für Cyclohexan-l,2-diyl oder 1,2-Phenylen.
- R¹: steht besonders bevorzugt für Wasserstoff, Amino oder Methyl.
- R²: steht besonders bevorzugt für jeweils durch Fluor und/oder Chlor substituiertes Methyl oder Ethyl.
- R³: steht besonders bevorzugt für Wasserstoff, Fluor, Chlor oder gegebenenfalls durch Fluor und/oder Chlor substituiertes Methyl.
- R⁴: steht besonders bevorzugt für Wasserstoff, Fluor oder Chlor.
- R⁵: steht besonders bevorzugt für Cyano, Carbamoyl, Thiocarbamoyl oder jeweils gegebenenfalls durch Fluor und/oder Chlor substituiertes Methyl oder Methoxy.
- R⁶: steht besonders bevorzugt für Wasserstoff oder Methyl.
- A: steht ganz besonders bevorzugt für jeweils gegebenenfalls durch Fluor, Chlor, Methyl, Ethyl, Methoxycarbonyl oder Ethoxycarbonyl substituiertes Ethan-1,2-diyl (Dimethylen) oder Propan-1,3-diyl (Trimethylen).
- R¹: steht ganz besonders bevorzugt für Methyl.
- R²: steht ganz besonders bevorzugt für Trifluormethyl.
- R³: steht ganz besonders bevorzugt für Wasserstoff, Chlor oder Methyl.
- R⁴: steht ganz besonders bevorzugt für Fluor oder Chlor.
- R⁵: steht ganz besonders bevorzugt für Cyano oder Thiocarbamoyl.
- R⁶: steht ganz besonders bevorzugt für Wasserstoff.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- oder Zwischenprodukte. Diese Restedefinitionen können untereinander, also auch zwischen den angegebenen bevorzugten Bereichen beliebig kombiniert werden.

Erfindungsgemäß bevorzugt sind diejenigen Verbindungen der Formel (1), bei welchen eine Kombination der vorstehend als bevorzugt aufgeführten Bedeutungen vorliegt.

Erfindungsgemäß besonders bevorzugt sind diejenigen Verbindungen der Formel (1), bei welchen eine Kombination der vorstehend als besonders bevorzugt aufgeführten Bedeutungen vorliegt.

Erfindungsgemäß ganz besonders bevorzugt sind diejenigen Verbindungen der Formel (I), bei welchen eine Kombination der vorstehend als ganz besonders bevorzugt aufgeführten Bedeutungen vorliegt.

Gesättigte oder ungesättigte Kohlenwasserstoffreste, wie Alkyl oder Alkenyl, sind - auch in Verbindung mit Heteroatomen, wie in Alkoxy - soweit möglich jeweils geradkettig oder verzweigt.

Gegebenenfalls substituierte Reste können einfach oder mehrfach substituiert sein, wobei bei Mehrfachsubstitution die Substituenten gleich oder verschieden sein können.

Die neuen substituierten Phenyluracile der allgemeinen Formel (I) weisen interessante biologische Eigenschaften auf. Sie zeichnen sich insbesondere durch starke herbizide Wirksamkeil aus.

Man erhält die neuen substituierten Phenyluracile der allgemeinen Formel (1), wenn man
Phenyluracile der allgemeinen Formel (II) 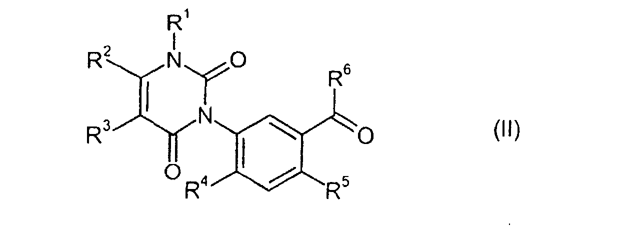 in welcher
R¹, R², R³, R⁴, R⁵ und R⁶ die oben angegebene Bedeutung haben,
mit zweifach nucleophilen Verbindungen der allgemeinen Formel (III) 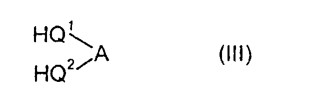 in welcher
A, Q¹ und Q² die oben angegebene Bedeutung haben,
gegebenenfalls in Gegenwart eines Reaktionshilfsmittels und gegebenenfalls in Gegenwart eines Verdünnungsmittels umsetzt,
und gegebenenfalls mit den so erhaltenen Verbindungen der allgemeinen Formel (1) Folgeumsetzungen im Rahmen der obigen Substituentendefinition nach üblichen Mcthoden durchrührt.

Die Verbindungen der allgemeinen Formel (1) können nach üblichen Methoden in andere Verbindungen der allgemeinen Formel (1) gemäß obiger Substituentendefinition umgewandelt werden, beispielsweise durch Aminierung oder Alkylierung (z.B. R¹: H → NH₂, H → CH₃), oder auch beispielsweise durch Umsetzung mit Hydrogensulfid (R⁵: CN → CSNH₂).

Die erfindungsgemäßen Verbindungen der allgemeinen Formel (1), in welchen R⁵ für Cyano steht, können prinzipiell auch gemäß folgendem Formelschema hergestellt werden: 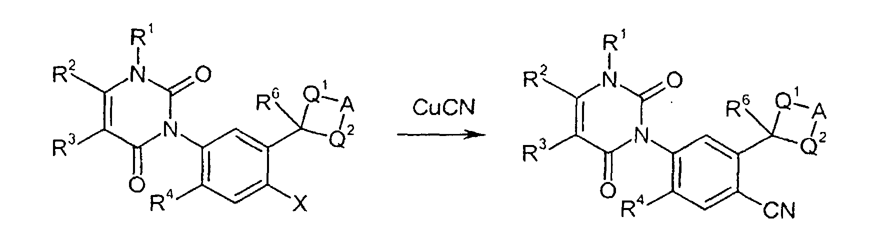 (X: Halogen, insbesondere Chlor oder Brom)

Verwendet man beispielsweise 1-(4-Cyano-2-chlor-5-formyl-phenyl)-3,6-dihydro-2,6-dioxo-3-methyl-4-trifluormethyl-1(2H)-pyrimidin und Propan-1,3-diol als Ausgangsstoffe, so kann der Reaktionsablauf beim erfindungsgemäßen Verfahren durch das folgende Formelschema skizziert werden:

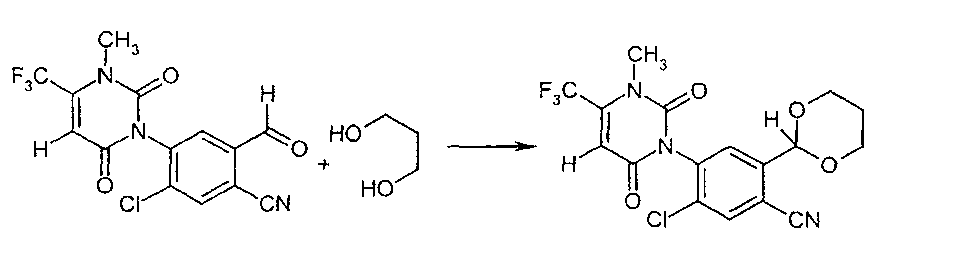

Die beim erfindungsgemäßen Verfahren zur Herstellung von Verbindungen der allgemeinen Formel (I) als Ausgangsstoffe zu verwendenden Phenyluracile sind durch die Formel (11) allgemein definiert. In der allgemeinen Foₙₙci (II) haben R¹, R². R³. R⁴, R⁵ und R⁶ vorzugsweise diejenigen Bedeutungen, die bereits oben im Zusammenhang mit der Beschreibung der erfindungsgemäßen Verbindungen der allgemeinen Formel (1) als bevorzugt, besonders bevorzugt oder ganz besonders bevorzugt für R¹, R², R³, R⁴, R⁵ und R⁶ angegeben worden sind.

Die Ausgangsstoffe der allgemeinen Formel (II) sind bekannt und/oder können nach an sich bekannten Verfahren hergestellt werden (vgl. WO-A-97/45418).

Die beim erfindungsgemäßen Verfahren zur Herstellung von Verbindungen der allgemeinen Formel (I) weiter als Ausgangsstoffe zu verwendenden zweifach nucleophilen Verbindungen sind durch die Formel (III) allgemein definiert. In der allgemeinen Formel (111) haben A, Q¹ und Q² vorzugsweise diejenigen Bedeutungen, die bereits oben im Zusammenhang mit der Beschreibung der erfindungsgemäßen Verbindungen der allgemeinen Formel (I) als bevorzugt, besonders bevorzugt oder ganz besonders bevorzugt für A, Q¹ und Q² angegeben worden sind.

Die Ausgangsstoffe der allgemeinen Formel (III) sind bekannte organische Synthesechemikalien.

Das erfindungsgemäße Verfahren zur Herstellung der Verbindungen der allgemeinen Formel (I) wird gegebenenfalls in Gegenwart eines Reaktionshilfsmittels durchgeführt. Es kommen hierbei vorzugsweise die üblichen Kondensationshilfsmittel in Betracht. Als Beispiel für ein besonders bevorzugtes Kondensationshilfsmittel sei Chlortrimethylsilan genannt.

Das erfindungsgemäßen Verfahren zur Herstellung der Verbindungen der allgemeinen Formel (I) wird gegebenenfalls unter Verwendung eines Verdünnungsmittels durchgeführt. Als Verdünnungsmittel zur Durchführung des erfindungsgemäßen Verfahrens kommen vor allem inerte organische Lösungsmittel in Betracht. Hierzu gehören insbesondere aliphalische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester, sowie Sulfoxide, wie Dimethylsulfoxid.

Die Reaktionstemperaturen können bei der Durchführung des erfindungsgemäßen Verfahrens in einem größeren Bereich variiert werden. Im allgemeinen arbeitet man bei Temperaturen zwischen 0°C und 150°C, vorzugsweise zwischen 10°C und 120°C.

Das erfindungsgemäße Verfahren wird im allgemeinen unter Normaldruck durchgeführt. Es ist jedoch auch möglich, das erfindungsgemäße Verfahren unter erhöhtem oder vermindertem Druck - im allgemeinen zwischen 0,1 bar und 10 bar - durchzuführen.

Zur Durchführung des erfindungsgemäßen Verfahrens werden die Ausgangsstoffe im allgemeinen in angenähert äquimolaren Mengen eingesetzt. Es ist jedoch auch möglich, eine der Komponenten in einem größeren Überschuß zu verwenden. Die Umsetzung wird im allgemeinen in einem geeigneten Verdünnungsmittel in Gegenwart eines Reaktionshilfsmittels durchgeführt und das Reaktionsgemisch wird im allgemeinen mehrere Stunden bei der erforderlichen Temperatur gerührt. Die Aufarbeitung wird nach üblichen Methoden durchgeführt (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemüßen Stoffe als totale oder selektive Herbizide wirken, hängt im wesentlichen von der angewendeten Menge ab. Die erfindungsgemäßen Wirkstoffe können z.B. bei den folgenden Pflanzen verwendet werden:

Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurca, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindemia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Soianum, So nehus, Sphenoclea, Stellaria, Taraxacum, Thlaspi, Trifolium, Urtica, Veronica, Viola, Xanthium.

Dikotyle Kulturen der Gattungen: Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.

Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Monokotyle Kulturen der Gattungen: Allium, Ananas, Asparagus, Avena, Hordeum, Oryza, Panicum, Saccharum, Secale, Sorghum, Triticale, Triticum, Zea.

Die Verwendung der erfindungsgeniäßen Wirkstoffe ist jedoch keineswegs auf diese Gattungen beschränkt, sondern erstreckt sich in gleicher Weise auch auf andere Pflanzen.

Die erfindungsgemäßen Wirkstoffe eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen der Formel (I) zeigen starke herbizide Wirksamkeit und ein breites Wirkungsspektrum bei Anwendung auf dem Boden und auf oberirdisehe Pflanxenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Melallphthalocyaninfarbstoffe und Spurennährstoffe wie Salze von Eisen, Mangan Bor, Kupfer, Kobalt, Molybdän und Zink verwendet werden.

Die Formulierungen enthallen im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Acetochlor, Acifluorfen(-sodium), Aclonifen, Alachlor, Alloxydim(-sodium), Ametryne, Amidochlor, Amidosulfuron, Anilofos, Asulam, Atrazine, Azafenidin, Azimsulfuron, Benazolin(-ethyl), Benfuresate, Bensulfuron(-methyl), Bentazon, Benzobicyclon, Benzofenap, Benzoylprop(-ethyl), Bialaphos, Bifenox, Bispyribac(sodium), Bromobutide, Bromofenoxim, Bromoxynil, Butachlor, Butroxydim, Butylate, Cafenstrole, Caloxydim, Carbetamide, Carfentrazone(-ethyl), Chlomethoxyfen, Chloramben, Chloridazon, Chlorimuron(-ethyl), Chlomitrofen, Chlorsulfuron, Chlortoluron, Cinidon(-ethyl), Cinmethylin, Cinosulfuron, Clefoxydim, Clethodim, Clodinafop(-propargyl), Clomazone, Clomeprop, Clopyralid, Clopyrasulfuron(-methyl), Cloransulam(-methyl), Cumyluron, Cyanazine, Cybutryne, Cycloate, Cyclosulfamuron, Cycloxydim, Cyhalofop(-butyl), 2,4-D, 2,4-DB, 2,4-DP, Desmedipham, Diallate, Dicamba, Diclofop(-methyl), Diclosulam, Diethatyl(-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epropodan, EPTC, Esprocarb, Ethalfluralin, Ethametsulfuron(-methyl), Ethofumesate, Ethoxyfen, Ethoxysulfuron, Etobenzanid, Fenoxaprop-(-P-ethyl), Fentrazamide, Flamprop(-isopropyl), Flamprop(-isopropyl-L), Flamprop(methyl), Flazasulfuron, Florasulam, Fluazifop(-P-butyl), Fluazolate, Flucarbazone, Flufenacet, Flumetsulam, Flumiclorac(-pentyl), Flumioxazin, Flumipropyn, Flumetsulam, Fluometuron, Fluorochloridone, Fluoroglycofen(-ethyl), Flupoxam, Flupropacil, Flurpyrsulfuron(-methyl, -sodium), Flurenol(-butyl), Fluridone, Fluroxypyr(meptyl), Flurprimidol, Flurtamone, Fluthiacet(-methyl), Fluthiamide, Fomesafen, Glufosinale(-ammonium), Glyphosate(-isopropylammonium), Halosafen, Haloxyfop-(-ethoxyethyl), Haloxyfop(-P-methyl), Hexazinone, lmazamethabenz(-methyl), Imazamethapyr, Imazamox, Imazapic, Imazapyr, Imazaquin, Imazethapyr, Imazosulfuron, lodosulfuron(-methyl, -sodium), loxynil, Isopropalin, Isoproturon, Isouron, Isoxaben, Isoxachlortole, Isoxaflutole, Isoxapyrifop, Lactofen, Lenacil, Linuron, MCPA, MCPP, Mefenacet, Mesotrione, Metamitron, Metazachlor, Methabenzthiazuron, Metobenzuron, Metobromuron, (alpha-)Metolachlor, Metosulam, Metoxuron, Metribuzin, Metsulfuron(-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziciomefone, Oxyfluorfen, Paraquat, Pelargonsäure, Pendimethalin, Pendralin, Pentoxazone, Phenmedipham, Piperophos, Pretilachlor, Primisulfuron(-methyl), Prometryn, Propachlor, Propanil, Propaquizafop, Propisochlor, Propyzamide, Prosulfocarb, Prosulfuron, Pyraflufen(-ethyl), Pyrazolate, Pyrazosulfuron(-ethyl), Pyrazoxyfen, Pyribenzoxim, Pyributicarb, Pyridate, Pyriminobac(methyl), Pyrithiobac(-sodium), Quinchlorac, Quinmerac, Quinoclamine, Quizalofop-(-P-ethyl), Quizalofop(-P-tefuryl), Rimsulfuron, Sethoxydim, Simazine, Simetryn, Sulcotrione, Sulfentrazone, Sulfometuron(-methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron(-methyl), Thiobencarb, Tiocarbazil, Tralkoxydim, Triallate, Triasulfuron, Tribenuron(-methyl), Triclopyr, Tridiphane, Trifluralin und Triflusulfuron.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsfomien wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

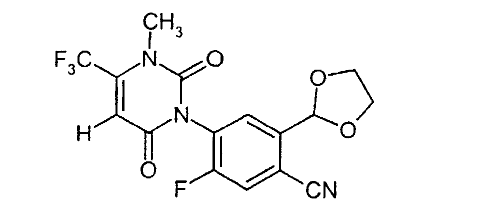

Eine Mischung aus 0,60 g (1,8 mMol) 1-(4-Cyano-2-fluor-5-fomyl-phenyl)-36-di-hydro-2,6-dioxo-3-methyl-4-trifluormethyl- 1 (2H)-pyrimidin, 1 ml Ethan-1,2-diol, 0,9 ml Chlortrimethylsilan und 20 ml Methylenchlorid wird 18 Stunden bei Raumtemperatur (ca. 20°C) gerührt. Anschließend wird mit Wasser verdünnt, die organische Phase abgetrennt, die wässrige Phase mit Methylenchlorid nachextrahiert, die vereinigten organischen Phasen mit Natriumsulfat getrocknet und filtriert. Vom Filtrat wird das Lösungsmittel im Wasserstrahlvakuum sorgfältig abdestilliert.

Man erhält 0,40 g ( 65% der Theorie) 1-[4-Cyano-2-fluor-5-(1,3-dioxolan-2-yl)-phenyl]-3,6-dihydro-2,6-dioxo-3-methyl-4-trifluormethyl-1(2H)-pyrimidin als festes Produkt.
¹H-NMR (D6-DMSO, δ: 6,58 ppm (s, 1H).

Analog zu Beispiel 1 sowie entsprechend der allgemeinen Beschreibung des erfindungsgemäßen Herstellungsverfahrens können beispielsweise auch die in der nachstehenden Tabelle 1 aufgeführten Verbindungen der allgemeinen Formel (I) hergestellt werden.

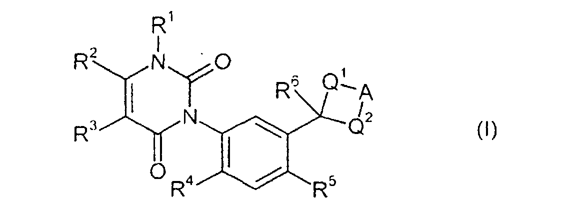

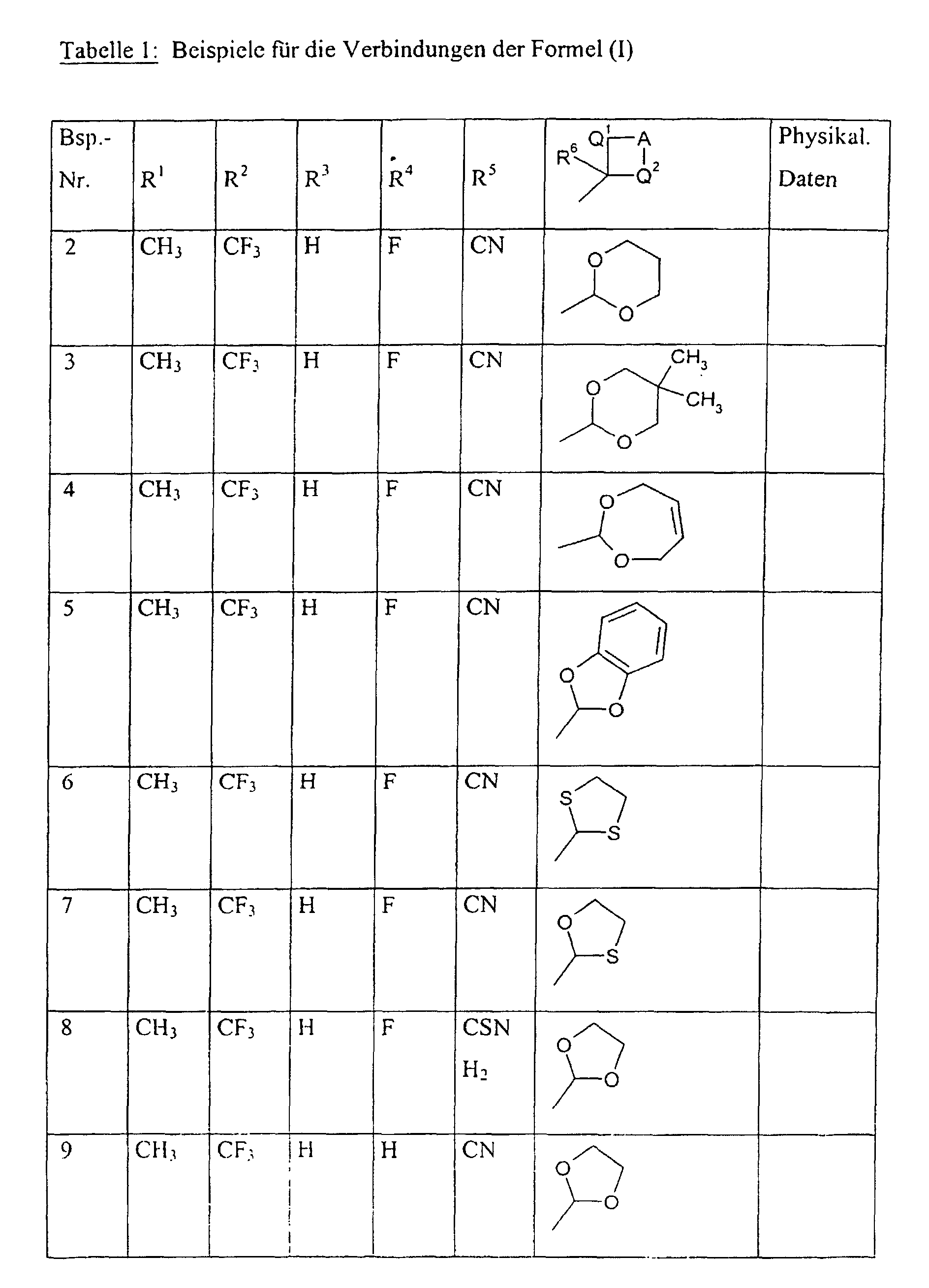

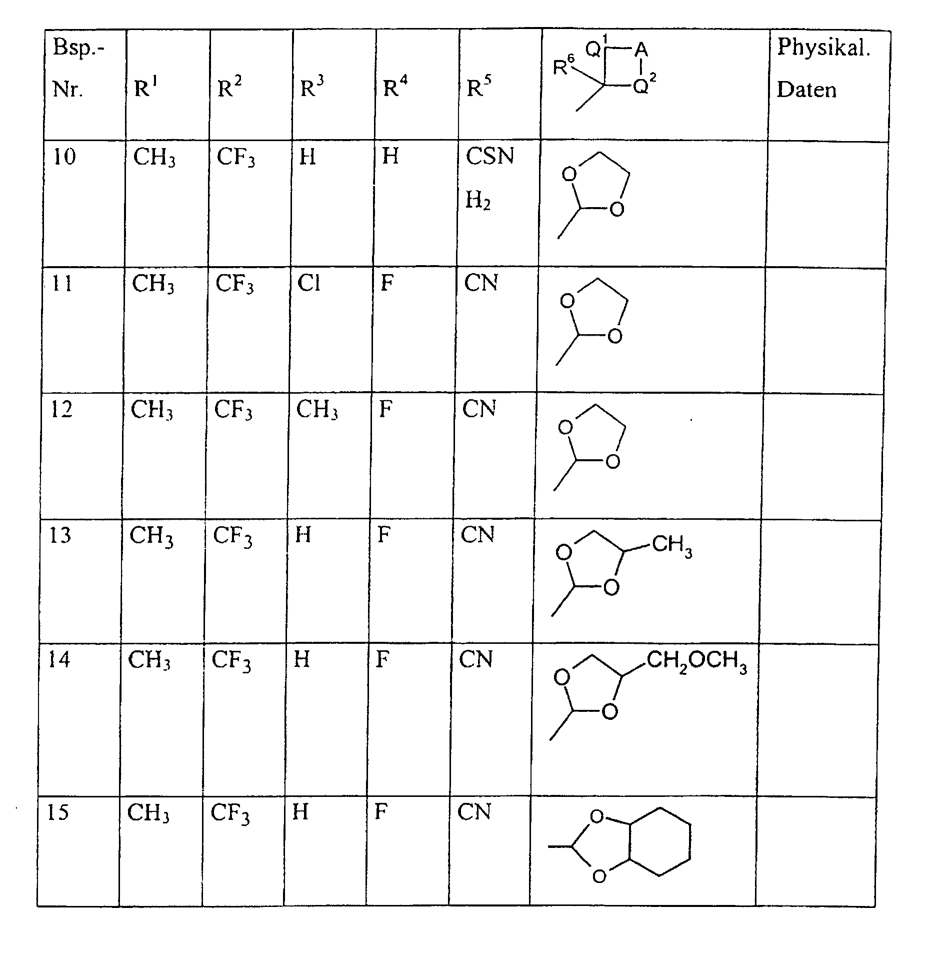

### Anwendungsbeispiele:

### Beispiel A

Pre-emergence-Test
- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach ca. 24 Stunden wird der Boden so mit der Wirkstoffzubereitung besprüht, daß die jeweils gewünschte Wirkstoffmenge pro Flächeneinheit ausgebracht wird. Die Konzentration der Spritzbrühe wird so gewählt, daß in 1000 Liter Wasser pro Hektar die jeweils gewünschte Wirkstoffmenge ausgebracht wird.

Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigt beispielsweise die Verbindung gemäß Herslellungsbeispiel 1 sehr starke Wirkung gegen Unkräuter.

### Beispiel B

Post-emergence-Test
- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 1000 1 Wasser/ha die jeweils gewünschten Wirkstoffinengen ausgebracht werden.

Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigt beispielsweise die Verbindung gemäß Herstellungsbeispiel 1 sehr starke Wirkung gegen Unkräuter.

## Patentansprüche

1. Substituierte Phenyluracile der allgemeinen Formel (I) 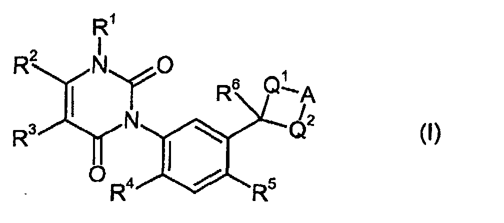 in welcher
A fiir jeweils gegebenenfalls durch Cyano, Halogen oder jeweils gegebenenfalls durch Halogen oder C₁-C₄-Alkoxy substituiertes C₁-C₄-Alkyl, C₁-C₄-Alkoxy-carbonyl oder C₂-C₄-Alkenyl substituiertes oder durch O (Sauerstoff) unterbrochenes Alkandiyl oder Alkendiyl mit jeweils 2 bis 5 Kohlenstoffatomen, oder für Cyclohexan-1,2-diyl oder 1,2-Phenylen steht,
Q¹ für O (Sauerstoff) oder S (Schwefel) steht,
Q² für O (Sauerstoff) oder S (Schwefel) steht,
R¹ für Wasserstoff, Amino oder gegebenenfalls durch Cyano, Halogen oder C₁-C₄-Alkoxy substituiertes Alkyl mit 1 bis 4 Kohlenstoffatomen steht,
R² fiir gegebenenfalls durch Halogen substituiertes Alkyl mit 1 bis 4 Kohlenstoffatomen steht,
R³ für Wasserstoff, Halogen oder gegebenenfalls durch Halogen substituiertes Alkyl mit 1 bis 4 Kohlenstoffatomen steht,
R⁴ für Wasserstoff, Cyano, Carbamoyl, Thiocarbamoyl oder Halogen steht,
R⁵ für Cyano, Carbamoyl, Thiocarbamoyl oder jeweils gegebenenfalls durch Halogen substituiertes Alkyl oder Alkoxy mit jeweils 1 bis 4 Kohlenstoffatomen steht, und
R⁶ für Wasserstoff oder Alkyl mit 1 bis 4 Kohlenstoffatomen steht.

2. Verbindungen gemäß Anspruch 1, **dadurch gekennzeichnet, daß**
A für jeweils gegebenenfalls durch Cyano, Fluor, Chlor, Brom, oder jeweils gegebenenfalls durch Fluor und/oder Chlor, Methoxy oder Ethoxy substituiertes Methyl, Ethyl, n- oder i-Propyl, Methoxy-carbonyl, Ethoxycarbonyl, n- oder i-Propoxycarbonyl, Ethenyl oder Propenyl substituiertes oder durch O (Sauerstoff) unterbrochenes Ethan-1,2-diyl (Dimethylen), Propan-1,3-diyl (Trimethylen), Ethen-1,2-diyl oder Propen-1,3-diyl, oder für Cyclohexan-1,2-diyl oder 1,2-Phenylen steht,
R¹ für Wasserstoff, Amino oder jeweils gegebenenfalls durch Cyano, Fluor, Chlor, Methoxy oder Ethoxy substituiertes Methyl, Ethyl, n-oder i-Propyl steht,
R² fiir jeweils gegebenenfalls durch Fluor und/oder Chlor substituiertes Methyl, Ethyl, n- oder i-Propyl steht,
R³ fiir Wasserstoff, Fluor, Chlor oder jeweils gegebenenfalls durch Fluor und/oder Chlor substituiertes Methyl oder Ethyl steht,
R⁴ für Wasserstoff, Cyano, Carbamoyl, Thiocarbamoyl, Fluor, Chlor oder Brom steht,
R⁵ für Cyano, Carbamoyl, Thiocarbamoyl oder jeweils gegebenenfalls durch Fluor und/oder Chlor substituiertes Methyl, Ethyl, Methoxy oder Ethoxy steht, und
R⁶ für Wasserstoff, Methyl oder Ethyl steht.

3. Verbindungen gemäß Anspruch 1 oder 2, **dadurch gekennzeichnet, daß**
A für jeweils gegebenenfalls durch Fluor, Chlor, Methyl, Ethyl, Methoxycarbonyl oder Ethoxycarbonyl substituiertes Ethan-1,2-diyl (Dimethylen), Propan-1,3-diyl (Trimethylen) oder Ethen-1,2-diyl, oder für Cyclohexan-1,2-diyl oder 1,2-Phenylen steht,
R¹ für Wasserstoff, Amino oder Methyl steht,
R² für jeweils durch Fluor und/oder Chlor substituiertes Methyl oder Ethyl steht,
R³ für Wasserstoff, Fluor, Chlor oder gegebenenfalls durch Fluor und/oder Chlor substituiertes Methyl steht,
R⁴ für Wasserstoff, Fluor oder Chlor steht,
R⁵ für Cyano, Carbamoyl, Thiocarbamoyl oder jeweils gegebenenfalls durch Fluor und/oder Chlor substituiertes Methyl oder Methoxy steht, und
R⁶ für Wasserstoff oder Methyl steht.

4. Verbindungen gemäß einem der Ansprüche 1 bis 3, **dadurch gekennzeichnet, daß**
A für jeweils gegebenenfalls durch Fluor, Chlor, Methyl, Ethyl, Methoxycarbonyl oder Ethoxycarbonyl substituiertes Ethan-1,2-diyl (Dimethylen), Propan-1,3-diyl (Trimethylen) steht,
R¹ für Methyl steht,
R² für Trifluormethyl steht,
R³ für Wasserstoff, Chlor oder Methyl steht,
R⁴ für Fluor oder Chlor steht,
R⁵ für Cyano oder Thiocarbamoyl steht, und
R⁶ für Wasserstoff steht.

5. Verbindungen gemäß einem der Ansprüche 1 bis 4, **dadurch gekennzeichnet, daß**
Q¹ für Sauerstoff (O) steht.

6. Verfahren zum Herstellen von Verbindungen gemäß einem der Ansprüche 1 bis 5, **dadurch gekennzeichnet, daß** man Phenyluracile der allgemeinen Formel (II) 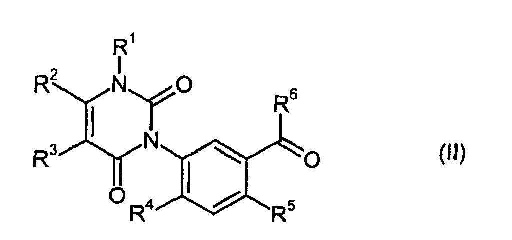 in welcher
R¹, R², R³, R⁴, R⁵ und R⁶ die in einem der Ansprüche 1 bis 4 angegebene Bedeutung haben,
mit zweifach nucleophilen Verbindungen der allgemeinen Formel (III) 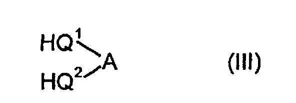 in welcher
A, Q¹ und Q² die in einem der Ansprüche 1 bis 5 angegebene Bedeutung haben,
gegebenenfalls in Gegenwart eines Reaktionshilfsmittels und gegebenenfalls in Gegenwart eines Verdünnungsmittels umsetzt,
und gegebenenfalls mit den so erhaltenen Verbindungen der allgemeinen Formel (I) Folgeumsetzungen im Rahmen der obigen Substituentendefinition nach üblichen Methoden durchführt.

7. Herbizide Mittel, **gekennzeichnet durch** den Gehalt mindestens einer Verbindung gemäß einem der Ansprüche 1 bis 5 und üblichen Streckmitteln.

8. Verwendung von mindestens einer Verbindung gemäß einem der Ansprüche 1 bis 5 zur Bekämpfung von unerwünschten Pflanzen.

## Claims

1. Substituted phenyuracils of the general formula (I) 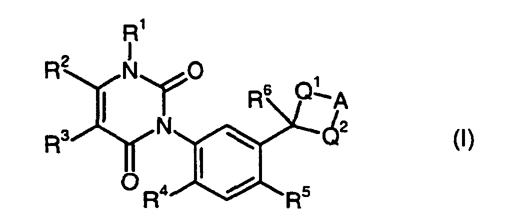 in which
A represents alkanediyl or alkenediyl, each of which has 2 to 5 carbon atoms and each of which is optionally substituted by cyano, halogen or by in each case optionally halogen- or C₁-C₄-alkoxy-substituted C₁-C₄-alkyl, C₁-C₄-alkoxy-carbonyl **or** C₂-C₄-alkenyl **or interrupted** by O (oxygen), or represents cyclohexane-1,2-diyl or 1,2-phenylene,
Q¹ represents O (oxygen) or S (sulphur),
Q² represents O (oxygen) or S (sulphur),
R¹ represents hydrogen, amino or optionally cyano-, halogen- or C₁-C₄-alkoxy-substituted alkyl having 1 to 4 carbon atoms,
R² represents optionally halogen-substituted alkyl having 1 to 4 carbon atoms,
R³ represents hydrogen, halogen or optionally halogen-substituted alkyl having 1 to 4 carbon atoms,
R⁴ represents hydrogen, cyano, carbamoyl, thiocarbamoyl or halogen,
R⁵ represents cyano, carbamoyl, thiocarbamoyl or in each case optionally halogen-substituted alkyl or alkoxy having in each case 1 to 4 carbon atoms, and
R⁶ represents hydrogen or alkyl having 1 to 4 carbon atoms.

2. Compounds according to Claim 1, **characterized in that**
A represents ethane-1,2-diyl (dimethylene), propane-1,3-diyl (trimethylene), ethene-1,2-diyl or propene-1,3-diyl, each of which is optionally substituted by cyano, fluorine, chlorine, bromine or by in each case optionally fluorine- and/or chlorine-, methoxy- or ethoxy-substituted methyl, ethyl, n- or i-propyl, methoxycarbonyl, ethoxycarbonyl, n- or i-propoxycarbonyl, ethenyl or propenyl or interrupted by O (oxygen), or represents cyclohexane-1,2-diyl or 1,2-phenylene,
R¹ represents hydrogen, amino or in each case optionally cyano-, fluorine-, chlorine-, methoxy- or ethoxy-substituted methyl, ethyl, n-or i-propyl,
R² represents in each case optionally fluorine- and/or chlorine-substituted methyl, ethyl, n- or i-propyl,
R³ represents hydrogen, fluorine, chlorine or in each case optionally fluorine- and/or chlorine-substituted methyl or ethyl,
R⁴ represents hydrogen, cyano, carbamoyl, thiocarbamoyl, fluorine, chlorine or bromine,
R⁵ represents cyano, carbamoyl, thiocarbamoyl or in each case optionally fluorine- and/or chlorine-substituted methyl, ethyl, methoxy or ethoxy, and
R⁶ represents hydrogen, methyl or ethyl.

3. Compounds according to Claim 1 or 2, **characterized in that**
A represents in each case optionally fluorine-, chlorine-, methyl-, ethyl-, methoxycarbonyl- or ethoxycarbonyl-substituted ethane-1,2-diyl (dimethylene), propane-1,3-diyl (trimethylene) or ethene-1,2-diyl, or represents cyclohexane-1,2-diyl or 1,2-phenylene,
R¹ represents hydrogen, amino or methyl,
R² represents in each case fluorine- and/or chlorine-substituted methyl or ethyl,
R³ represents hydrogen, fluorine, chlorine or optionally fluorine- and/or chlorine-substituted methyl,
R⁴ represents hydrogen, fluorine or chlorine,
R⁵ represents cyano, carbamoyl, thiocarbamoyl or in each case optionally fluorine- and/or chlorine-substituted methyl or methoxy, and
R⁶ represents hydrogen or methyl.

4. Compounds according to any of Claims 1 to 3, **characterized in that**
A represents in each case optionally fluorine-, chlorine-, methyl-, ethyl-, methoxycarbonyl- or ethoxycarbonyl-substituted ethane-1,2-diyl (dimethylene), propane-1,3-diyl (trimethylene),
R¹ represents methyl,
R² represents trifluoromethyl,
R³ represents hydrogen, chlorine or methyl,
R⁴ represents fluorine or chlorine,
R⁵ represents cyano or thiocarbamoyl, and
R⁶ represents hydrogen.

5. Compounds according to any of Claims 1 to 4, **characterized in that**
Q¹ represents oxygen (O).

6. Process for preparing compounds according to any of Claims 1 to 5, **characterized in that** phenyluracils of the general formula (II) 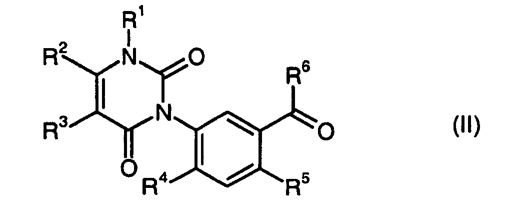 in which
R¹, R², R³, R⁴, R⁵ and R⁶ are each as defined in one of Claims 1 to 4,
are reacted with dinucleophilic compounds of the general formula (III) 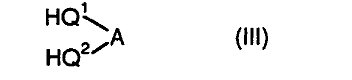 in which
A, Q¹ and Q² are each as defined in one of Claims 1 to 5,
if appropriate in the presence of a reaction auxiliary and if appropriate in the presence of a diluent,
and subsequent reactions within the scope of the above definition of substituents are carried out, if appropriate, by customary methods on the resulting compounds of the general formula (I).

7. Herbicidal compositions, **characterized in that** they comprise at least one compound according to any of Claims 1 to 5 and customary extenders.

8. Use of at least one compound according to any of Claims 1 to 5 for controlling undesirable plants.

## Revendications

1. Phényluraciles substitués de la formule générale (I) : 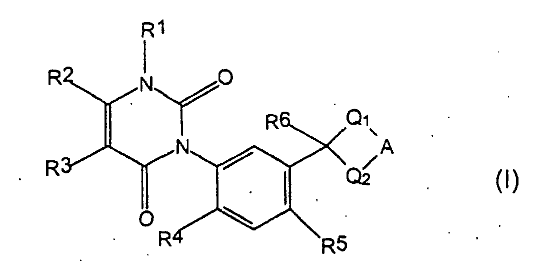 dans laquelle :
A représenté un radical alcanediyle ou alcènediyle, interrompu par l'atome O (oxygène) ou chaque fois le cas échéant substitué par le radical cyano, un atome d'halogène ou un radical alcoyle en C₁-C₄, (alcoxy en C₁-C₄)carbonyle ou alcényle en C₂-C₄, chaque fois le cas échéant substitué par un atome d'halogène ou un radical alcoxy en C₁-C₄, avec chaque fois 2 à 5 atomes de.carbone, ou représente le radical cyclohexane-1,2-diyle ou 1,2-phénylène ;
Q¹ représente l'atome O (oxygène) ou S (soufre),
Q² représente l'atome O (oxygène) ou S (soufre),
R¹ représente l'atome d'hydrogène, le radical amino ou un radical alcoyle le cas échéant substitué par le radical cyano, un atome d'halogène ou un radical alcoxy en C₁-C₄, avec 1 à 4 atomes de carbone ;
R² représente un radical alcoyle le cas échéant substitué par un atome d'halogène, ayant 1 à 4 atomes de carbone ;
R³ représente l'atome d'hydrogène, un atome d'halogène ou un radical alcoyle le cas échéant substitué par un atome d'halogène, ayant 1 à 4 atomes de carbone ;
R⁴ représente l'atome d'hydrogène, le radical cyano, carbamoyle, thiocarbamoyle ou un atome d'halogène ;
R⁵ représente le radical cyano, carbamoyle, thiocarbamoyle ou un radical alcoyle ou alcoxy, chaque fois le cas échéant substitué par un atome d'halogène, ayant 1 à 4 atomes de carbone, et
R⁶ représente l'atome d'hydrogène ou un radical alcoyle ayant 1 à 4 atomes de carbone.

2. Composés suivant la revendication 1, **caractérisés en ce que** :
A représente le radical éthane-1,2-diyle (diméthylène), propane-1,3-diyle (triméthylène), éthène-1,2-diyle ou propène-1,3-diyle, interrompu par l'atome O (oxygène) ou chaque fois le cas échéant substitué par le radical cyano, l'atome de fluor, de chlore, de brome ou le radical méthyle, éthyle, n- ou i-propyle, méthoxycarbonyle, éthoxycarbonyle, n- ou i-propoxycarbonyle, éthényle ou propényle, chaque fois le cas échéant substitué par l'atome de fluor et/ou de chlore, le radical méthoxy ou éthoxy, ou représente le radical cyclohexane-1,2-diyle ou 1,2-phénylène ;
R¹ représente l'atome d'hydrogène, le radical amino ou le radical méthyle, éthyle, n- ou i-propyle, chaque fois le cas échéant substitué par le radical cyano, l'atome de fluor, de chlore, le radical méthoxy ou éthoxy ;
R² représente le radical méthyle, éthyle, n- ou i-propyle, chaque fois le cas échéant substitué par l'atome de fluor et/ou de chlore ;
R³ représente l'atome d'hydrogène, de fluor, de chlore ou le radical méthyle ou éthyle, chaque fois le cas échéant substitué par l'atome de fluor et/ou de chlore ;
R⁴ représente l'atome d'hydrogène, le radical cyano, carbamoyle, thiocarbamoyle, l'atome de fluor, de chlore ou de brome ;
R⁵ représente le radical cyano, carbamoyle, thiocarbamoyle ou le radical méthyle, éthyle, méthoxy ou éthoxy, chaque fois le cas échéant substitué par l'atome de fluor et/ou de chlore, et
R⁶ représente l'atome d'hydrogène, le radical méthyle ou éthyle.

3. Composés suivant la revendication 1 ou 2, **caractérisés en ce que** :
A représente le radical éthane-1,2-diyle (diméthylène), propane-1,3-diyle (triméthylène), ou éthène-1,2-diyle chaque fois le cas échéant substitué par l'atome de fluor, de chlore, le radical méthyle, éthyle, méthoxycarbonyle, éthoxycarbonyle, ou représente le radical cyclohexane-1,2-diyle ou 1,2-phénylène ;
R¹ représente l'atome d'hydrogène, le radical amino ou le radical méthyle ;
R² représente le radical méthyle ou éthyle, chaque fois substitué par l'atome de fluor et/ou de chlore ;
R³ représente l'atome d'hydrogène, de fluor, de chlore ou le radical méthyle le cas échéant substitué par l'atome de fluor et/ou de chlore ;
R⁴ représente l'atome d'hydrogène, de fluor ou de chlore ;
R⁵ represente le radical cyano, carbamoyle, thiocarbamoyle ou le radical méthyle ou méthoxy, chaque fois le cas échéant substitué par l'atome de fluor et/ou de chlore, et
R⁶ représente l'atome d'hydrogène ou le radical méthyle.

4. Composés suivant l'une quelconque des revendications 1 à 3, **caractérisés en ce que** :
A représente le radical éthane-1,2-diyle (diméthylène), propane-1,3-diyle (triméthylène), chaque fois le cas échéant substitué par l'atome de fluor, de chlore, le radical méthyle, éthyle, méthoxycarbonyle, éthoxycarbonyle ;
R¹ représente le radical méthyle ;
R² représente le radical trifluorométhyle ;
R³ représente l'atome d'hydrogène, de chlore ou le radical méthyle ;
R⁴ représente l'atome de fluor ou de chlore ;
R⁵ représente le radical cyano ou thiocarbamoyle, et
R⁶ représente l'atome d'hydrogène.

5. Composés suivant l'une quelconque des revendications 1 à 4, **caractérisés en ce que** :
Q¹ représente l'atome O (oxygène).

6. Procédé de préparation de composés suivant l'une quelconque des revendications 1 à 5, **caractérisé en ce que** l'on fait réagir des phényluraciles de la formule générale (II) : 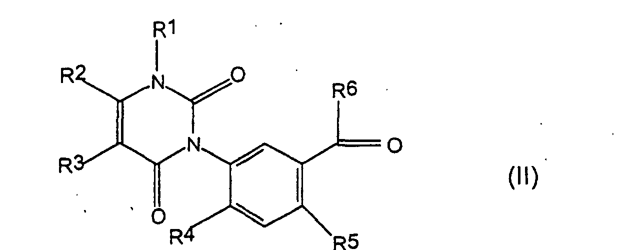 dans laquelle :
R¹, R², R³, R⁴, R⁵ et R⁶ ont la signification indiquée dans l'une des revendications 1 à 4,
avec des composés binucléophiles de la formule générale (III) : 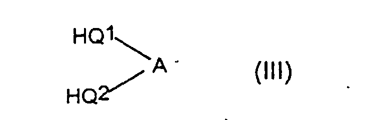
dans laquelle A, Q¹ et Q² ont la signification indiquée dans l'une des revendications 1 à 5,
le cas échéant en présence d'un auxiliaire de réaction et le cas échéant, en présence d'un agent de dilution,
et le cas échéant, on réalise avec les composés ainsi obtenus de la formule générale (I), des réactions ultérieures dans le cadre des définitions ci-dessus de substituant, selon des procédés usuels.

7. Agent herbicide, **caractérisé par** une teneur en au moins un composé suivant l'une quelconque des revendications 1 à 5 et les diluants usuels.

8. Utilisation d'au moins un composé suivant l'une quelconque des revendications 1 à 5, pour lutter contre des végétaux non désirés.
